# DEMANDE DE BREVET EUROPEEN

(11) **EP 1 424 062 A1**
(43) Date de publication de la demande: **02.06.2004**
(21) Numéro de dépôt: 03292531.5
(22) Date de dépôt: 13.10.2003
(51) Int. Cl.: A61K 7/42

(54) **Composition filtrante contenant au moins un dérivé du dibenzoylmethane et au moins dérivé de 3-(2-azacycloalkylidène)-1,3-dihydro-indol-2-one ; procédé de photostabilisation**

(30) Priorité: 29.11.2002 FR 0215057
(71) Demandeur: L'OREAL, 75008 Paris (FR)
(72) Inventeur: Rozot, Roger, 77400 Lagny/Marne (FR); Deflandre, André, 60560 Orry la Ville (FR)
(74) Mandataire: Miszputen, Laurent

(57) **Abrégé**

L'invention concerne une composition cosmétique ou dermatologique, à usage topique, en particulier pour la photoprotection de la peau et des cheveux, caractérisée par le fait qu'elle comprend au moins dans un support cosmétiquement acceptable:
(a) au moins un filtre UV du type dérivé du dibenzoylméthane et
(b) au moins un dérivé de 3-(2-azacycloalkylidene)-1,3-dihydro-indol-2-one dans une quantité efficace.

L'invention concerne également un procédé pour améliorer la stabilité d'au moins un dérivé du dibenzoylméthane vis-à-vis du rayonnement UV consistant à associer au dit dérivé de dibenzoylméthane une quantité efficace d'un dérivé de 3-(2-azacycloalkylidene)-1,3-dihydro-indol-2-one.

## Description

L'invention concerne une composition cosmétique ou dermatologique, à usage topique, en particulier pour la photoprotection de la peau et des cheveux, caractérisée par le fait qu'elle comprend au moins dans un support cosmétiquement acceptable:
(b) au moins un filtre UV du type dérivé du dibenzoylméthane et
(b) au moins un dérivé de 3-(2-azacycloalkylidene)-1,3-dihydro-indol-2-one dans une quantité efficace de dérivé de dibenzoylméthane.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

A cet égard, une famille de filtres UV-A particulièrement intéressante est actuellement constituée par les dérivés du dibenzoylméthane, et notamment le 4-ter-butyl-4'-méthoxydibenzoyl méthane, qui présentent en effet un fort pouvoir d'absorption intrinsèque. Ces dérivés du dibenzoylméthane, qui sont maintenant des produits bien connus en soi à titre de filtres actifs dans les UV-A, sont notamment décrits dans les demandes de brevets français FR-A-2326405 et FR-A-2440933, ainsi que dans la demande de brevet européen EP-A-0114607 ; le 4-ter-butyl- 4'-méthoxydibenzoyl méthane est par ailleurs actuellement proposé à la vente sous la dénomination commerciale de "PARSOL 1789" par la Société HOFFMANN LAROCHE.

Malheureusement, il se trouve que les dérivés du dibenzoylméthane sont des produits relativement sensibles au rayonnement ultraviolet (surtout UV-A), c'est-à-dire, plus précisément, qu'ils présentent une fâcheuse tendance à se dégrader plus ou moins rapidement sous l'action de ce dernier. Ainsi, ce manque substantiel de stabilité photochimique des dérivés du dibenzoylméthane face au rayonnement ultraviolet auquel ils sont par nature destinés à être soumis, ne permet pas de garantir une protection constante durant une exposition solaire prolongée, de sorte que des applications répétées à intervalles de temps réguliers et rapprochés doivent être effectuées par l'utilisateur pour obtenir une protection efficace de la peau contre les rayons UV.

La photostabilisation des dérivés du dibenzoylméthane vis-à-vis du rayonnement UV constitue, à ce jour, un problème qui n'a pas encore été résolu de manière complètement satisfaisante.

Or, la Demanderesse vient maintenant de découvrir, de façon surprenante, qu'en associant aux dérivés du dibenzoylméthane mentionnés ci-dessus une quantité efficace d'un dérivé de 3-(2-azacycloalkylidene)-1,3-dihydro-indol-2-one, il était possible d'améliorer de manière substantielle et remarquable, la stabilité photochimique (ou photostabilité) de ces mêmes dérivés du dibenzoylméthane.

Cette découverte, essentielle, est à la base de la présente invention.

Ainsi, conformément à l'un des objets de la présente invention, il est maintenant proposé une nouvelle composition cosmétique ou dermatologique, à usage topique, caractérisée par le fait qu'elle comprend au moins, dans un support cosmétiquement acceptable :
(a) au moins un filtre UV du type dérivé du dibenzoylméthane et
(b) au moins un dérivé de 3-(2-azacycloalkylidene)-1,3-dihydro-indol-2-one dans une quantité efficace.

La présente invention a également pour objet un procédé pour améliorer la stabilité d'au moins un dérivé du dibenzoylméthane vis-à-vis du rayonnement UV consistant à associer au dit dérivé de dibenzoylméthane une quantité efficace d'au moins un dérivé de 3-(2-azacyctoalkylidene)-1,3-dihydro-indol-2-one.

Par quantité efficace d'un dérivé de 3-(2-azacycloalkyfidene)-1,3-dihydro-indol-2-one au sens de la présente l'invention, on entend une quantité suffisante pour obtenir une amélioration notable et significative de la photostabilité du ou des dérivés du dibenzoylméthane de la composition cosmétique photoprotectrice. Cette quantité minimale en agent photostabilisant à mettre en oeuvre, qui peut varier selon la nature du support cosmétiquement acceptable retenu pour la composition, peut être déterminée sans aucune difficulté au moyen d'un test classique de mesure de photostabilité.

Par dérivé de 3-(2-azacycloalkylidene)-1,3-dihydro-indol-2-one au sens de la présente l'invention, on entend tout composé simple, oligomère ou polymère possédant sur la chaîne des greffons contenant le groupe 3-(2-azacycloalkylidene)-1,3-dihydro-indol-2-one.

La présente invention a également enfin pour objet l'utilisation d'au moins un dérivé de 3-(2-azacycloalkylidene)-1,3-dihydro-indol-2-one dans la préparation d'une composition cosmétique ou dermatologique comprenant au moins un dérivé du dibenzoylméthane dans le but d'améliorer la stabilité vis-à-vis des rayons UV dudit dérivé de dibenzoylméthane.

D'autres caractéristiques, aspects et avantages de la présente invention apparaîtront à la lecture de la description détaillée qui va suivre.

Les dérivés de 3-(2-azacycloalkylidene)-1,3-dihydro-indol-2-one conformes à l'invention sont choisis de préférence parmi ceux répondant à la formule générale (I) suivante : 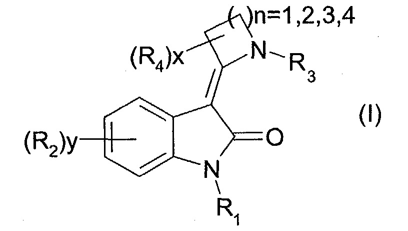 dans laquelle :
**R**_{**1**} **et R**_{**3**} sont identiques ou différents et peuvent être :
   - un atome d'hydrogène,
   - un groupe alkyle en C₁-C₂₂ linéaire ou ramifié, saturé ou insaturé, éventuellement substitué par un ou plusieurs groupes A₁,
   - un cycle de 4 à 7 atomes, saturé ou insaturé, contenant éventuellement au moins un hétéroatome parmi N, O et S, éventuellement accolé à un autre cycle, éventuellement substitué par un ou plusieurs groupes A₁, ces cycles pouvant comporter une ou plusieurs fonctions carbonyle ou thiocarbonyle,
   - un des groupes C(=NR₅)R'₅, C(=NR₅)NR'₅R"₅, COR₅, CSR₅, COOR₅, CONR₅R'₅, CSNR₅R'₅, SO₂R₅, SO₂NR₅R'₅ avec R₅, R'₅ et R"₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂.
**R**_{**2**} **et R**_{**4**} sont identiques ou différents et peuvent être :
   - un atome d'hydrogène,
   - un groupe alkyle en C₁-C₂₂ linéaire ou ramifié, saturé ou insaturé éventuellement, substitué par un ou plusieurs groupes A₁,
   - un cycle de 4 à 7 atomes, saturé ou insaturé, contenant éventuellement au moins un hétéroatome parmi N, O et S, éventuellement substitué par un ou plusieurs groupes A₁, ce cycle pouvant accolé à un autre cycle ou aux cycles de la structure 3-(2-azacycloalkylidene)-1,3-dihydro-indol-2-one objet de l'invention, ces cycles pouvant comporter une ou plusieurs fonctions carbonyle ou thiocarbonyle, ce cycle pouvant également être chargé positivement comme imidazolium, pyridinium, pyrazolium, triazolium,
   - un halogène comme F, Cl, Br,
   - un des groupes CF₃, CN, OR₅, SR₅, NR₅R'₅, C(=NR₅)R'₅, C(=NR₅)NR'₅R"₅, NR₅C(=NR'₅)NR"₅R"'₅, COR₅, CSR₅, COOR₅, CONR₅R'₅, NR₅COR'₅, NR₅CONR'₅R"₅, CSNR₅R'₅, SO₂NR₅R'₅, NR₅SO₂R'₅, SO₂R₅, NR₅R'₅R"₅R"'₅⁺ avec R₅, R'₅, R"₅ et R"'₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂ ;
**A**_{**1**} peut être :
   - un halogène comme F, Cl, Br,
   - un groupe alkyle en C₁-C₂₂ linéaire ou ramifié, saturé ou insaturé, éventuellement substitué par un ou plusieurs groupes A₂,
   - un des groupes CF₃, CN, OR₅, SR₅, NR₅R'₅, C(=NR₅)R'₅, C(=NR₅)NR'₅R"₅, NR₅C(=NR'₅)NR"₅R'"₅, COR₅, CSR₅, COOR₅, CONR₅R'₅, NR₅COR'₅, NR₅CONR'₅R''₅, CSNR₅R'₅, SO₂NR₅R'₅, NR₅SO₂R'₅, SO₂R₅, SiR₅R'₅R"₅, SiR₅(OSiR'₅R"₅R"'₅)OSiR'₅R"₅R'"₅, NR₅R'₅R"₅R"'₅⁺ avec R₅, R'₅, R"₅ et R"'₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂
   - un cycle de 4 à 7 atomes, saturé ou insaturé, contenant éventuellement au moins un hétéroatome parmi N, O et S, éventuellement substitué par un ou plusieurs groupes A₂, ce cycle pouvant accolé à un autre cycle ou aux cycles de la structure 3-(2-azacycloalkylidene)-1,3-dihydro-indol-2-one objet de l'invention, ces cycles pouvant comporter une ou plusieurs fonctions carbonyle ou thiocarbonyle, ce cycle pouvant également être chargé positivement comme imidazolium, pyridinium, pyrazolium, triazolium,
**A**_{**2**} peut être :
   - un halogène comme F, Cl, Br,
   - un groupe alkyle en C₁-C₂₂ linéaire ou ramifié, saturé ou insaturé,
   - un des groupes CF₃, CN, OR, SR, NRR', C(=NR)R', C(=NR)NR'R", NRC(=NR')NR"R"', COR, CSR, COOR, CONRR', NRCOR', NRCONR'R", CSNRR', SO₂NRR', NRSO₂R', SO₂R, SiRR'R", SiR(OSiR'R"R"')OSiR'R"R"', NRR'R"R'"⁺ avec R, R', R" et R"' identiques ou différents désignant l'hydrogène ou un radical alkyle en C₁-C₂₂, linéaire ou ramifié.
y vaut 1,2,3 ou 4 ;
x vaut de 1 à 2n+2.

Comme exemples de groupes alkyle en C₁-C₂₂ linéaires ou ramifiés, saturés ou insaturés, on peut citer : méthyle, isopropyle, éthyle-2-hexyle, *ter*-butyle, éthylène, propylène. Cette liste n'est pas limitative.

Comme exemples d'hétérocycles, on peut citer : pyrrole, furanne, thiophène, imidazole, oxazole, thiazole, pyrazole, isoxazole, isothiazole, triazole, oxadiazole, thiadiazole, tétrazole, pyridine, pipéridine, pyrimidine, pipérazine, pyridazine, pyrazine, triazine, morpholine, pyrrolidine, thiazolidine. Cette liste n'est pas limitative.

Comme exemples de cycles carbonés à 4, 5, 6 ou 7 atomes, saturés ou insaturés, on peut citer: cyclobutyle, cyclopentyle, cyclohexyle, cyclohexényle, phényle ou cycloheptyle. Cette liste n'est pas limitative.

Les dérivés de 3-(2-azacycloalkylidene)-1,3-dihydro-indol-2-one de formule (I) conformes à l'invention peuvent être préparés selon un procédé de synthèse mettant en oeuvre un acétal de lactame et un dérivé de 1,3-dihydro-indol-2-one à température ambiante dans l'éther anhydre selon le schéma réactionnel suivant cité dans les articles ci-dessus :

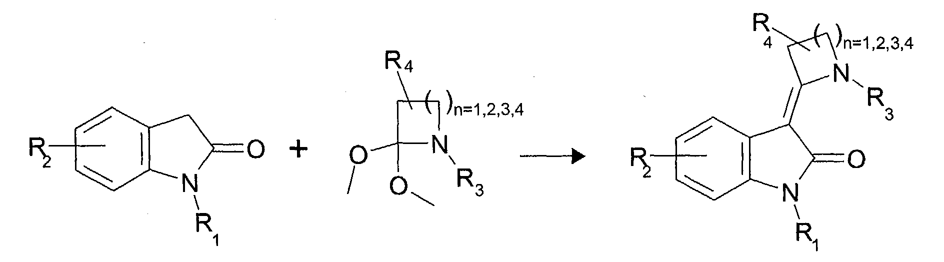

De nombreux dérivés lactame et 1,3-dihydro-indol-2-one sont disponibles chez la plupart des fournisseurs de produits chimiques comme par exemple :

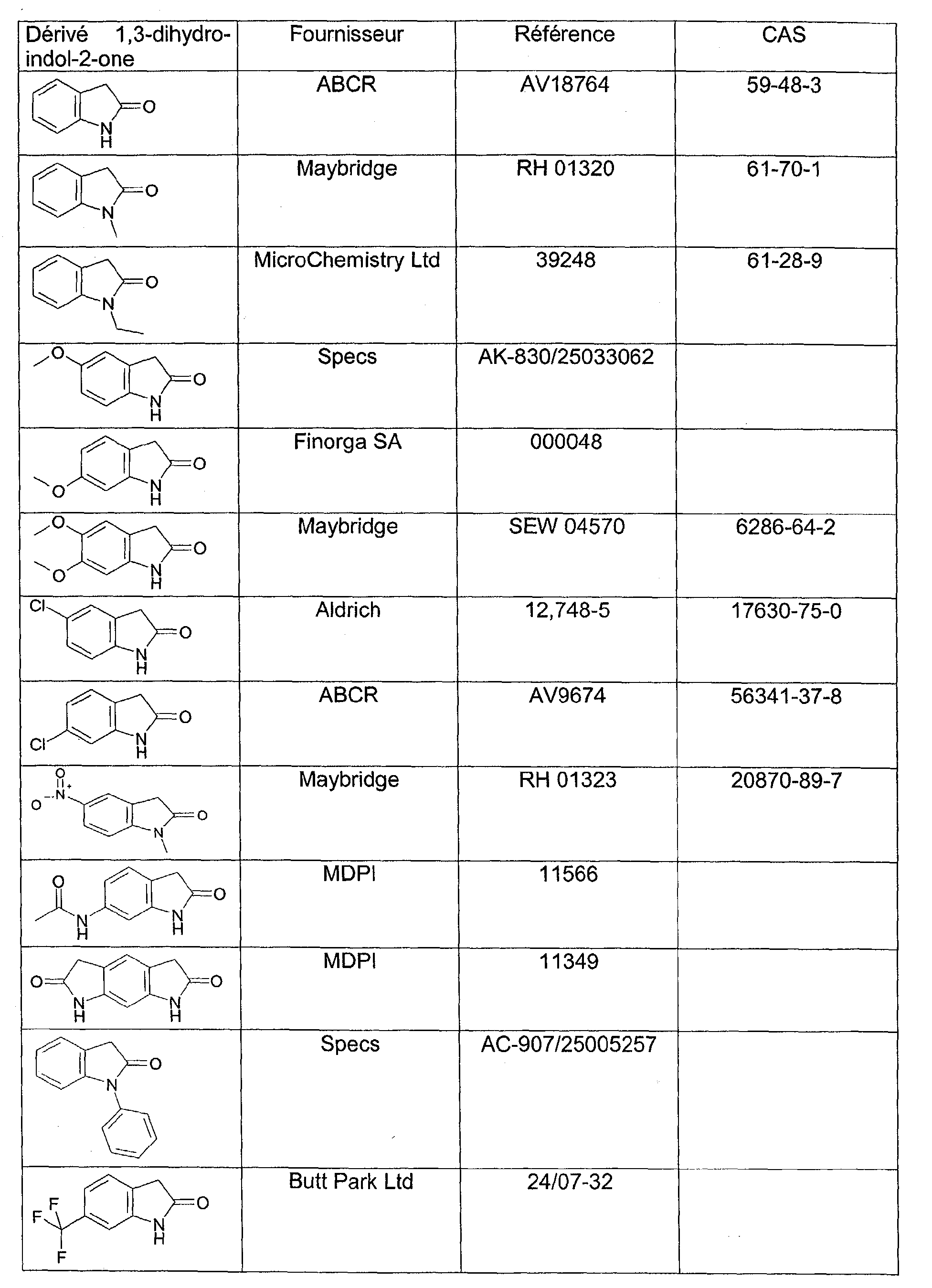

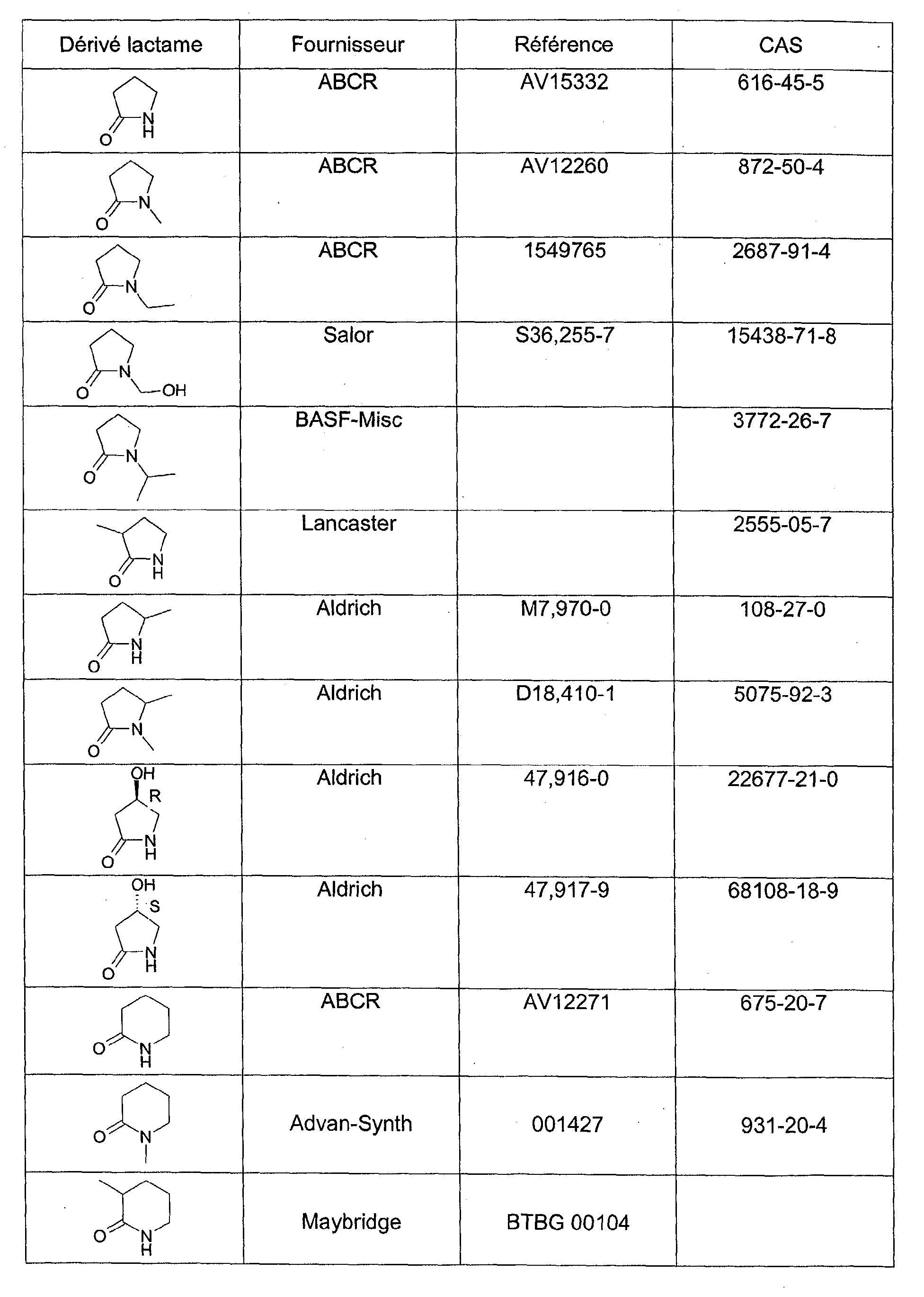

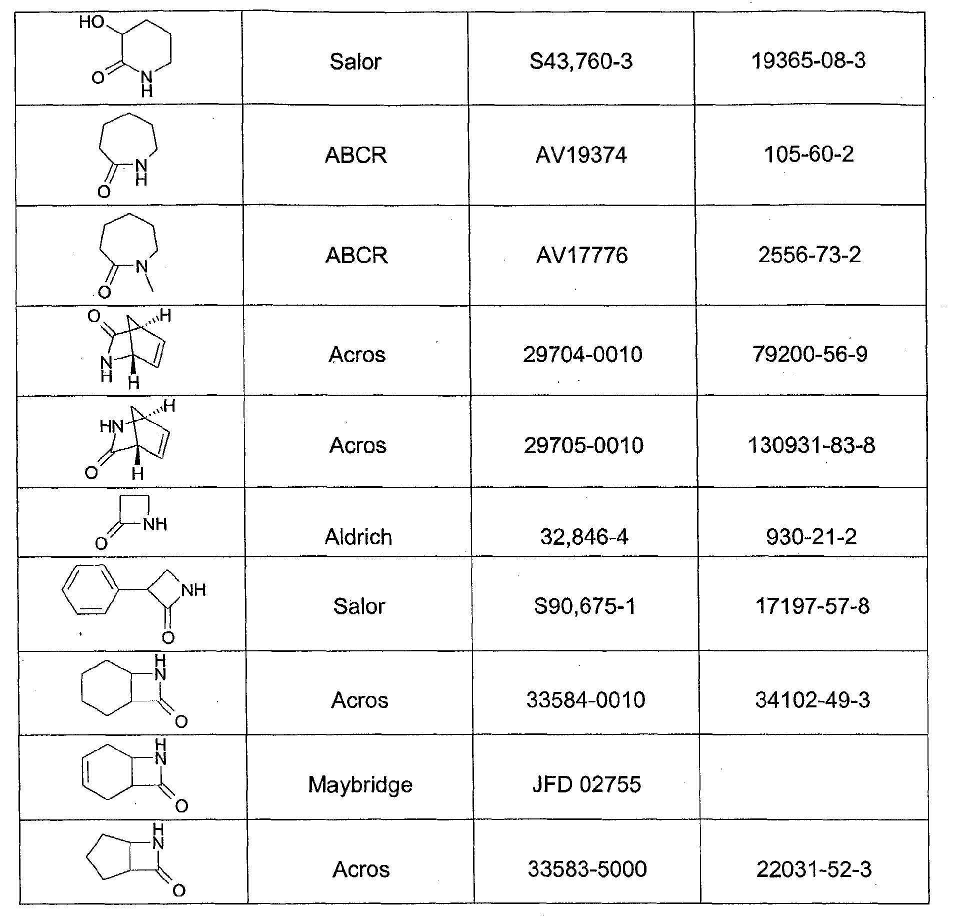

Les acétals de lactame peuvent être obtenus à partir des lactames selon le schéma réactionnel suivant cité dans les articles Tetrahedron Letters (1994), 35 (18), 2951 - 2954 et Journal of Organic Chemistry (1984), 49, 3659 - 3660 :

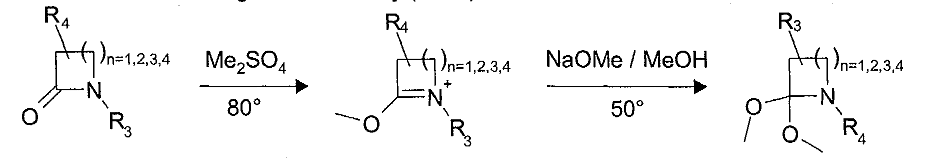

Selon le lactame de départ, des étapes de protection puis de déprotection de certains groupes fonctionnels comme OH ou NH, bien connues de l'homme de l'art, peuvent être nécessaires.

Parmi les composés de formule (I) préférentiels , on peut citer ceux de formule (II) ou (III) suivantes : 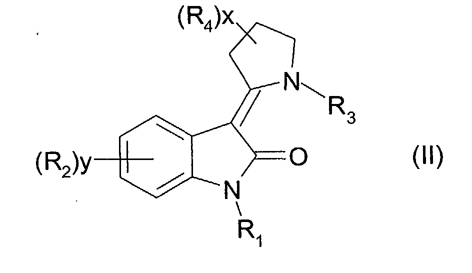 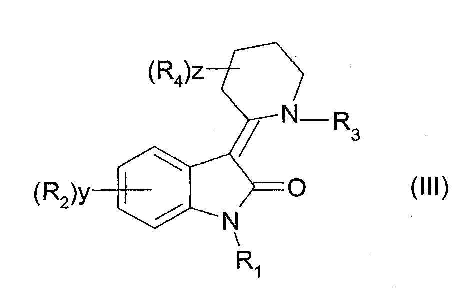 dans lesquelles :
R₁ et R₃, identiques ou différents, désignent l'hydrogène, un radical alkyle en C₁-C₈, linéaire ou ramifié, saturé ou insaturé, le groupe phényle ou un radical COR₅ ou SO₂R₅ avec R₅ désignant un radical alkyle en C₁-C₈ linéaire ou ramifié, saturé ou insaturé, ou le groupe phényle ;
R₂ désigne l'hydrogène ; un radical alkyle en C₁-C₈, linéaire ou ramifié, saturé ou insaturé ; le groupe phényle ; un des radicaux OR₅, NR₅R'₅, NR₅COR'₅, COOR₅ et CONR₅R'₅ avec R₅ et R'₅, identiques ou différents, désignant l'hydrogène, un radical alkyle en C₁-C₈ linéaire ou ramifié, saturé ou insaturé, ou le groupe phényle ; le radical CF₃ ;
R₄ désigne l'hydrogène, un radical alkyle en C₁-C₈, linéaire ou ramifié, saturé ou insaturé, le groupe phényle ou un radical OR₅ avec R₅ désignant l'hydrogène, un radical alkyle en C₁-C₈ linéaire ou ramifié, saturé ou insaturé, ou le groupe phényle ; x, y et z valent 1,2, 3 ou 4.

Parmi les dérivés de 3-(2-azacycloalkylidene)-1,3-dihydro-indol-2-one de formule (I) on citera encore plus particulièrement ceux de formule (II) où :
R₂ désigne l'hydrogène, OR₅, NR₅COR'₅ ou NR₅R'₅ avec R₅ et R'₅, identiques ou différents, désignant l'hydrogène, un radical alkyle en C₁-C₈ linéaire ou ramifié, saturé ou insaturé, ou le groupe phényle ;
R₄ désigne l'hydrogène ;
R₁ et R₃, identiques ou différents, désignent l'hydrogène, un radical alkyle en C₁-C₈, linéaire ou ramifié, saturé ou insaturé, ou le groupe phényle et encore plus particulièrement ceux décrits dans le tableau suivant, disponibles commercialement chez Specs/Biospecs :

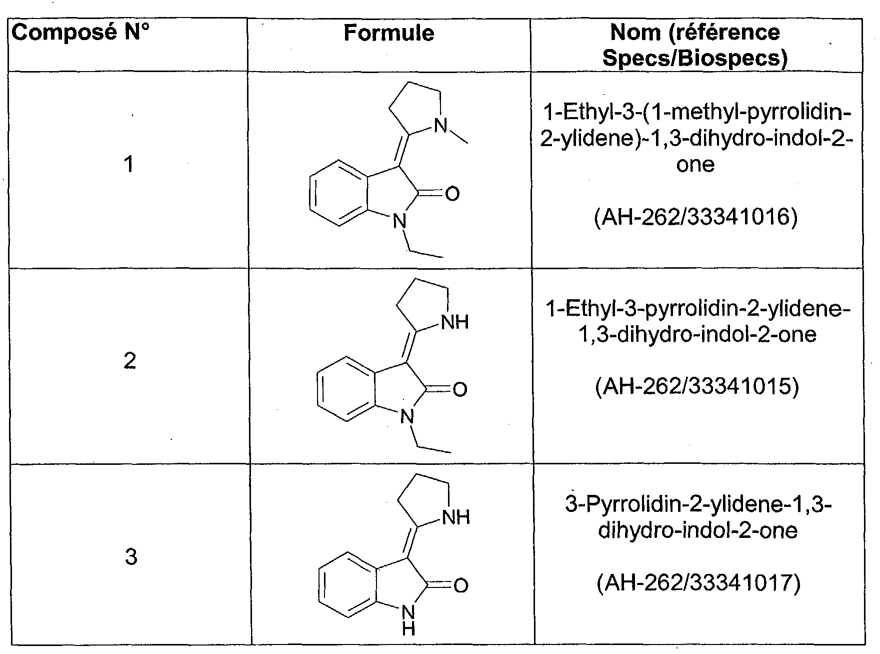

Les dérivés de 3-(2-azacycloalkylidene)-1,3-dihydro-indol-2-one conformes à l'invention sont présents de préférence dans la composition de l'invention dans des proportions allant de 0,01 % à 20% en poids, et plus préférentiellement de 0,1 à 10% en poids par rapport au poids total de la composition.

Comme indiqué précédemment, les dérivés du dibenzoylméthane destinés à être photostabilisés dans le cadre de la présente invention sont des produits déjà bien connus en soi et décrits notamment dans les documents FR2326405, FR2440933 et EP0114607 précités, documents dont les enseignements sont, pour ce qui touche à la définition même de ces produits, totalement inclus à titre de références dans la présente description.

Selon la présente invention, on peut bien entendu mettre en oeuvre un ou plusieurs dérivés du dibenzoylméthane.

Parmi les dérivés du dibenzoylméthane rentrant particulièrement bien dans le cadre de la présente invention, on peut notamment citer, de manière non limitative :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane

Parmi les dérivés du dibenzoylméthane mentionnés ci-dessus, on préfère tout particulièrement, selon la présente invention, mettre en oeuvre le 4-(terbutyl)-4'-méthoxydibenzoylméthane, notamment celui proposé à la vente sous la dénomination commerciale de "PARSOL 1789" par la Société HOFFMANN LAROCHE, ce filtre répondant donc à la formule développée suivante (ou à une de ses formes tautomères) :

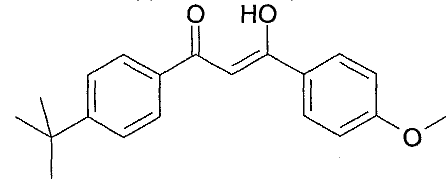

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante (ou à une de ses formes tautomères) :

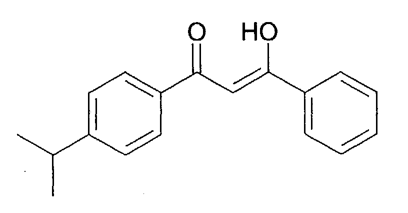

Le ou les dérivés du dibenzoylméthane peuvent être présents dans les compositions conformes à l'invention à des teneurs qui varient de préférence de 0,01 % à 20 % en poids, et plus préférentiellement de 0,1 à 10% en poids par rapport au poids total de la composition.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCl :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β,β'-diphénylacrylate :

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF, Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF, Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK , Benzylidene Camphor Sulfonic Acid fabriqué sous le nom « MEXORYL SL» par CHIMEX, Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

### Dérivés de la triazine :

Anisotriazine vendu sous le nom commercial «TINOSORB S » par CIBA GEIGY,
Ethylhexyl triazone vendu notamment sous le nom commercial «UVINUL T150 » par BASF,
2,4,6-tris-(4'-amino benzalmalonate de diisobutyle)-s- triazine,
Diethylhexyl Butamido Triazone vendu sous le nom commercial « UVASORB HEB » par SIGMA 3V,

### Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le produit Polysilicone-15 vendu sous la dénomination commerciale « PARSOL SLX » par HOFFMANN LA ROCHE

### Dérivés de 4,4-diarylbutadiène :

- 1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino)-6-(2-ethylhexyl)-imino-1,3,5-triazine vendu sous le nom Uvasorb K2A par Sigma 3V ;
et leurs mélanges.

Les filtres UV organiques complémentaires préférentiels sont choisis parmi :
- Ethylhexyl Salicylate,
- Ethylhexyl Methoxycinnamate
- Octocrylene,
- Phenylbenzimidazole Sulfonic Acid,
- Benzophenone-3,
- Benzophenone-4,
- Benzophenone-5,
- 2-(4-diethylamino-2-hydroxybenzoyl)-benzoate de n-hexyle.
- 4-Methylbenzylidene camphor,
- Terephthalylidene Dicamphor Sulfonic Acid,
- Disodium Phenyl Dibenzimidazole Tetra-sulfonate,
- la 2,4,6-tris-(4'-amino benzalmalonate de diisobutyle)-s-triazine
- Anisotriazine,
- Ethylhexyl triazone,
- Diethylhexyl Butamido Triazone,
- Methylène bis-Benzotriazolyl Tetramethylbutylphénol
- Drometrizole Trisiloxane
- Polysilicone-15
- 1,1-dicarboxy (2,2'-diméthyl-propyl)-4,4-diphénylbutadiène
- 2,4-bis-[5-1 (diméthylpropyl)benzoxazol-2-yl-(4-phenyl)-imino)-6-(2-ethylhexyl)-imino-1,3,5-triazine et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les,filtres UV complémentaires conformes à l'invention sont généralement présents dans les compositions selon l'invention dans des proportions allant de 0,1 à 20 % en poids par rapport au poids total de la composition, et de préférence allant de 0,2 à 15 % en poids par rapport au poids total de la composition.

Les compositions cosmétiques selon l'invention peuvent contenir en outre des agents de bronzage et/ou de brunissage artificiel de la peau (agents autobronzants) tels que la dihydroxyacétone (DHA).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les polymères acryliques réticulés comme les Carbomer, les polymères réticulés acrylates/C10-C30alkylacrylates du type Pemulen ou le polyacrylate-3 vendu sous le nom VISCOPHOBE DB 1000 par Amerchol ; les polyacrylamides tels que l'émulsion polyacrylamide, C13-C14 isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Bien entendu, l'homme de l'art veillera à choisir le ou les éventuels composés complémentaires cités ci-dessus et/ou leurs quantités de manière telle que les propriétés avantageuses attachées intrinsèquement compositions conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, d'une huile, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, d'huile solaire, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un objet de l'invention est l'utilisation d'une composition telle que définie précédemment pour la fabrication d'une composition cosmétique ou dermatologique destinées à la protection de la peau et/ou des cheveux contre le rayonnement ultraviolet, en particulier le rayonnement solaire.

Un autre objet de la présente invention réside dans un procédé pour améliorer la stabilité d'au moins un dérivé du dibenzoylméthane vis-à-vis du rayonnement UV consistant à associer au dit dérivé de dibenzoylméthane une quantité efficace d'au moins un dérivé de 3-(2-azacycloalkylidene)-1,3-dihydro-indol-2-one tel que défini ci-dessus.

Un autre objet de la présente invention consiste en l'utilisation d'un dérivé de 3-(2-azacycloalkylidene)-1,3-dihydro-indol-2-one tel que défini ci-dessus dans la préparation d'une composition cosmétique ou dermatologique comprenant au moins un filtre UV du type dérivé du dibenzoylméthane dans le but d'améliorer la stabilité vis-à-vis des rayons UV dudit dérivé du dibenzoylméthane.

Des exemples concrets, mais nullement limitatifs, illustrant l'invention, vont maintenant être donnés.

### EXEMPLES

### EXEMPLE 1

Dans cet exemple, on a étudié la photostabilité du 4-(terbutyl)-4'-méthoxydibenzoylméthane (filtre solaire "PARSOL 1789" de chez HOFFMANN LA ROCHE) en présence d'un composé photostabilisant selon l'invention à savoir :
1-Ethyl-3-(1-methvl-pyrrolidin-2-ylidene)-1,3-dihydro-indol-2-one (composé 1)

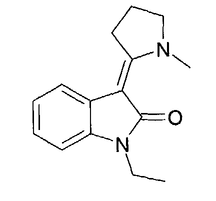

Ce composé peut être acheté chez Specs/Biospecs sous la référence AH-262/33341016 ou être obtenu selon le procédé de synthèse défini ci-dessus à partir de 1-Ethyl-1,3-dihydro-indol-2-one (cas : 61-28-9) et de 1-Méthyl-pyrrolidin-2-one (cas : 872-50-4).

A titre comparatif, on a étudié la photostabilité de ce même filtre en l'absence de ce composé photostabilisant..

On prépare une solution à 0,2% (pourcentage massique) de Parsol 1789 et 0,8% de 1-Ethyl-3-(1-methyl-pyrrolidin-2-ylidene)-1,3-dihydro-indol-2-one (composé 1) dans un mélange contenant 20% de Finsolv TN + 80% d'éthanol.

Après évaporation, sur un support, de l'éthanol contenu dans 10 µl de la solution décrite ci dessus, on obtient un film huileux d'épaisseur moyenne -20 µm contenant 1% de Parsol 1789 + 4% de composé photostabilisant. Ce film est exposé pendant une heure au simulateur solaire (19 mW/cm² en UVA et 1,2 mW/cm² en UVB).

Les compositions de ces deux formules (F0-F1) étaient ainsi les suivantes (% en poids par rapport au poids total de la formule) :

| Composition | Composé photostabilisant | Support |
|---|---|---|
| F0 (comparatif) | 0 % | Support commun* |
| F1 (invention) | 4 % | Support commun* |

| | | |
|---|---|---|
| *la composition du support commun était elle-même la suivante (% en poids par rapport au poids total de la formule) : - 4-terbutyl-4'-méthoxy-dibenzoylméthane (PARSOL 1789) 1 % - Alkyl benzoate en C₁₂-C₁₅ (Finsolv TN) qsp 100 % | | |

La photostabilité du Parsol 1789 dans ces formulations a été quantifiée par dosage HPLC du filtre résiduel.

Le taux de filtre résiduel après irradiation s'exprime mathématiquement par le rapport entre la concentration en filtre mesurée dans l'échantillon irradié et la concentration initiale de ce filtre dans l'échantillon avant irradiation.

Les résultats obtenus ont été les suivants :

| **Compositions** | **PARSOL 1789 résiduel Après 1 heures d'irradiation** |
|---|---|
| F0 (comparatif) | 61 % |
| F1 (invention) | 99 % |

Ces résultats mettent clairement en évidence l'effet de photostabilisation remarquable apporté par le 1-Ethyl-3-(1-methyl-pyrrolidin-2-ylidene)-1,3-dihydro-indol-2-one (composé 1) conforme à l'invention sur le 4-(terbutyl)-4'-méthoxy-dibenzoylméthane.

### EXEMPLE 2

### 1-Ethyl-3-pyrrolidin-2-ylidene-1,3-dihydro-indol-2-one (composé 2)

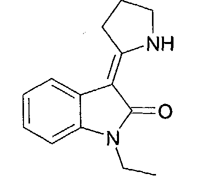

Ce composé peut être acheté chez Specs/Biospecs sous la référence AH-262/33341015 ou être obtenu selon le procédé de synthèse défini ci-dessus à partir de 1-Ethyl-1,3-dihydro-indol-2-one (cas : 61-28-9) et de 1-Pyrrolidin-2-one (cas : 616-45-5).

On obtient un effet photostabilisant sur le 4-(terbutyl)-4'-méthoxy-dibenzoylméthane comparable à celui de l'exemple 1

### EXEMPLE 3

### 3-Pyrrolidin-2-ylidene-1,3-dihydro-indol-2-one (composé 3)

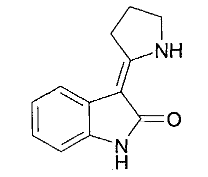

Ce composé peut être acheté chez Specs/Biospecs sous la référence AH-262/33341017 ou être obtenu selon le procédé de synthèse défini ci-dessus à partir de 1,3-Dihydroindol-2-one (cas : 59-48-3) et de 1-Pyrrolidin-2-one (cas : 616-45-5). On obtient un effet photostabilisant sur le 4-(terbutyl)-4'-méthoxy-dibenzoylméthane comparable à celui de l'exemple 1

## Revendications

1. Composition cosmétique ou dermatologique, à usage topique, **caractérisée par le fait qu'**elle comprend au moins, dans un support cosmétiquement acceptable :
(a) au moins un filtre UV du type dérivé du dibenzoylméthane et
(b) une quantité efficace d'un dérivé de 3-(2-azacycloalkylidene)-1,3-dihydro-indol-2-one.

2. Composition selon la revendication 1, où le dérivé de 3-(2-azacycloalkylidene)-1,3-dihydro-indol-2-one répond à la formule (I) suivante : 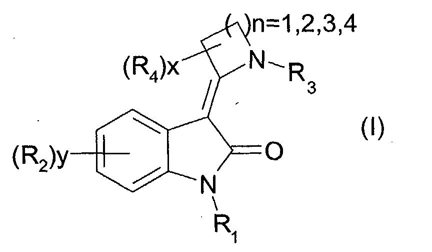 dans laquelle :
**R**_{**1**} **et R**_{**3**} sont identiques ou différents et peuvent être :
- un atome d'hydrogène,
- un groupe alkyle en C₁-C₂₂ linéaire ou ramifié, saturé ou insaturé, éventuellement substitué par un ou plusieurs groupes A₁,
- un cycle de 4 à 7 atomes, saturé ou insaturé, contenant éventuellement au moins un hétéroatome parmi N, O et S, éventuellement accolé à un autre cycle, éventuellement substitué par un ou plusieurs groupes A₁, ces cycles pouvant comporter une ou plusieurs fonctions carbonyle ou thiocarbonyle,
- un des groupes C(=NR₅)R'₅, C(=NR₅)NR'₅R"₅, COR₅, CSR₅, COOR₅, CONR₅R'₅, CSNR₅R'₅, SO₂R₅, SO₂NR₅R'₅ avec R₅, R'₅ et R"₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂..
**R**_{**2**} **et R**_{**4**} sont identiques ou différents et peuvent être :
- un atome d'hydrogène,
- un groupe alkyle en C₁-C₂₂ linéaire ou ramifié, saturé ou insaturé éventuellement, substitué par un ou plusieurs groupes A₁,
- un cycle de 4 à 7 atomes, saturé ou insaturé, contenant éventuellement au moins un hétéroatome parmi N, O et S, éventuellement substitué par un ou plusieurs groupes A₁, ce cycle pouvant accolé à un autre cycle ou aux cycles de la structure 3-(2-azacycloalkylidene)-1,3-dihydro-indol-2-one objet de l'invention, ces cycles pouvant comporter une ou plusieurs fonctions carbonyle ou thiocarbonyle, ce cycle pouvant également être chargé positivement comme imidazolium, pyridinium, pyrazolium, triazolium,
- un halogène comme F, Cl, Br,
- un des groupes CF₃, CN, OR₅, SR₅, NR₅R'₅, C(=NR₅)R'₅, C(=NR₅)NR'₅R"₅, NR₅C(=NR'₅)NR"₅R"'₅, COR₅, CSR₅, COOR₅, CONR₅R'₅, NR₅COR'₅, NR₅CONR'₅R"₅, CSNR₅R'₅, SO₂NR₅R'₅, NR₅SO₂R'₅, SO₂R₅, NR₅R'₅R"₅R"'₅⁺ avec R₅, R'₅, R"₅ et R"'₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂ ;
**A**_{**1**} peut être :
- un halogène comme F, Cl, Br,
- un groupe alkyle en C₁-C₂₂ linéaire ou ramifié, saturé ou insaturé, éventuellement substitué par un ou plusieurs groupes A₂,
- un des groupes CF₃, CN, OR₅, SR₅, NR₅R'₅, C(=NR₅)R'₅, C(=NR₅)NR'₅R"₅, NR₅C(=NR'₅)NR"₅R"'₅, COR₅, CSR₅, COOR₅, CONR₅R'₅, NR₅COR'₅, NR₅CONR'₅R"₅, CSNR₅R'₅, SO₂NR₅R'₅, NR₅SO₂R'₅, SO₂R₅, SiR₅R'₅R"₅, SiR₅(OSiR'₅R"₅R"'₅)OSiR'₅R"₅R"'₅, NR₅R'₅R"₅R"'₅⁺ avec R₅, R'₅, R"₅ et R"'₅ identiques ou différents désignant l'hydrogène, un radical alkyle en C₁-C₂₂, linéaire ou ramifié ou un cycle de 4 à 7 atomes, pouvant contenir au moins un hétéroatome parmi N, O, S, isolé ou accolé à un autre cycle, ces cycles pouvant comporter une ou plusieurs fonctions carbonyle ou thiocarbonyle, le radical alkyle ou lesdits cycles étant saturés ou non et éventuellement substitués par au moins un substituant A₂
- un cycle de 4 à 7 atomes, saturé ou insaturé, contenant éventuellement au moins un hétéroatome parmi N, O et S, éventuellement substitué par un ou plusieurs groupes A₂, ce cycle pouvant accolé à un autre cycle ou aux cycles de la structure 3-(2-azacycloalkylidene)-1,3-dihydro-indol-2-one objet de l'invention, ces cycles pouvant comporter une ou plusieurs fonctions carbonyle ou thiocarbonyle, ce cycle pouvant également être chargé positivement comme imidazolium, pyridinium, pyrazolium, triazolium,
**A**_{**2**} peut être :
- un halogène comme F, CI, Br,
- un groupe alkyle en C₁-C₂₂ linéaire ou ramifié, saturé ou insaturé,
- un des groupes CF₃, CN, OR, SR, NRR', C(=NR)R', C(=NR)NR'R", NRC(=NR')NR"R"', COR, CSR, COOR, CONRR', NRCOR', NRCONR'R", CSNRR', SO₂NRR', NRSO₂R', SO₂R, SiRR'R", SiR(OSiR'R"R"')OSiR'R"R"', NRR'R"R"'⁺ avec R, R', R" et R"' identiques ou différents désignant l'hydrogène ou un radical alkyle en C₁-C₂₂, linéaire ou ramifié.
y vaut 1,2,3 ou 4 ;
x vaut de 1 à 2n+2.

3. Composition selon la revendication 2, où le composé de formule (I) est choisi parmi ceux de formule (II) ou (III) suivantes : 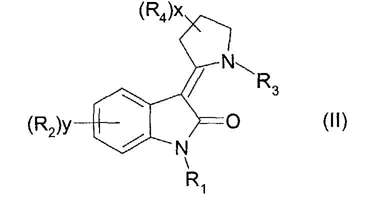 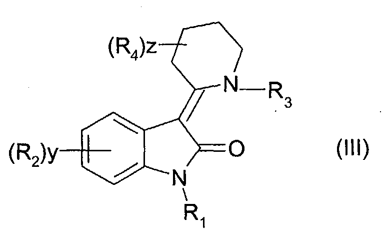 dans lesquelles :
R₁ et R₃, identiques ou différents, désignent l'hydrogène, un radical alkyle en C₁-C₈, linéaire ou ramifié, saturé ou insaturé, le groupe phényle ou un radical COR₅ ou SO₂R₅ avec R₅ désignant un radical alkyle en C₁-C₈ linéaire ou ramifié, saturé ou insaturé, ou le groupe phényle ;
R₂ désigne l'hydrogène ; un radical alkyle en C₁-C₈, linéaire ou ramifié, saturé ou insaturé ; le groupe phényle ; un des radicaux OR₅, NR₅R'₅, NR₅COR'₅, COOR₅ et
CONR₅R'₅ avec R₅ et R'₅, identiques ou différents, désignant l'hydrogène, un radical alkyle en C₁-C₈ linéaire ou ramifié, saturé ou insaturé, ou le groupe phényle ; le radical CF₃ ;
R₄ désigne l'hydrogène, un radical alkyle en C₁-C₈, linéaire ou ramifié, saturé ou insaturé, le groupe phényle ou un radical OR₅ avec R₅ désignant l'hydrogène, un radical alkyle en C₁-C₈ linéaire ou ramifié, saturé ou insaturé, ou le groupe phényle ; x, y et z valent 1,2, 3 ou 4.

4. Composition selon la revendication 3, où les composés de formule (II) sont choisis parmi ceux pour lesquels :
R₂ désigne l'hydrogène, OR₅, NR₅COR'₅ ou NR₅R'₅ avec R₅ et R'₅, identiques ou différents, désignant l'hydrogène, un radical alkyle en C₁-C₈ linéaire ou ramifié, saturé ou insaturé, ou le groupe phényle ;
R₄ désigne l'hydrogène ;
R₁ et R₃, identiques ou différents, désignent l'hydrogène, un radical alkyle en C₁-C₈, linéaire ou ramifié, saturé ou insaturé, ou le groupe phényle.

5. Composition selon la revendication 4, où les composés de formule (II) sont choisis parmi les composés suivants :
1-Ethyl-3-(1-methyl-pyrrolidin-2-ylidene)-1,3-dihydro-indol-2-one
1-Ethyl-3-pyrrolidin-2-ylidene-1,3-dihydro-indol-2-one
Pyrrolidin-2-ylidene-1 ,3-dihydro-indol-2-one.

6. Composition selon l'une quelconque des revendications 1 à 5, **caractérisée par le fait que** le composé de formule (I) est présent dans la composition à une teneur allant de 0,01 à 20 % en poids, par rapport au poids total de la composition, de préférence allant de 0,1 % à 10 % en poids, par rapport au poids total de la composition.

7. Composition selon l'une quelconque des revendications 1 à 6, **caractérisée par le fait que** le dérivé de dibenzoylméthane est choisi parmi :
- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

8. Composition selon la revendication 7, **caractérisée par le fait que** le dérivé de dibenzoylméthane est le 4-(terbutyl)-4'-méthoxy-dibenzoylméthane.

9. Composition selon la revendication 7, **caractérisée par le fait que** le dérivé de dibenzoylméthane est le 4-isopropyl-dibenzoylméthane.

10. Composition selon l'une quelconque des revendications 1 à 9, dans laquelle le dérivé du dibenzoylméthane est présent dans la composition à une teneur allant de 0,01 à 20 % en poids, par rapport au poids total de la composition, de préférence allant de 0,1 % à 10 % en poids, par rapport au poids total de la composition.

11. Composition selon l'une quelconque des revendications 1 à 10, où le support cosmétiquement acceptable se présente sous la forme d'une émulsion de type huile-dans-eau ou eau-dans-huile.

12. Composition selon l'une quelconque des revendications 1 à 11, **caractérisée par le fait qu'**elle comprend en outre un ou plusieurs filtres organiques ou inorganiques complémentaires actifs dans l'UV-A et/ou UV-B.

13. Composition selon la revendication 12 **caractérisée par le fait que** lesdits filtres organiques complémentaires sont choisis parmi les anthranilates; les dérivés cinnamiques ; les dérivés salicyliques, les dérivés du camphre ; les dérivés de triazine; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres; les dimères dérivés d'α-alkylstyrène; les 4,4-diarylbutadiènes et leurs mélanges.

14. Composition selon la revendication 13, **caractérisée par le fait que** lesdits filtres organiques complémentaires sont choisis parmi
Ethylhexyl Salicylate,
Ethylhexyl Methoxycinnamate
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

15. Composition selon la revendication 12, **caractérisée par le fait que** les filtres complémentaires inorganiques sont des pigments ou des nanopigments d'oxydes métalliques, enrobés ou non.

16. Composition selon la revendication 15, **caractérisée par le fait que** lesdits pigments ou nanopigments sont choisis parmi les oxydes de titane, de zinc, de fer, de zirconium, de cérium et leurs mélanges, enrobés ou non.

17. Composition selon l'une quelconque des revendications 1 à 16, **caractérisée par le fait qu'**elle comprend en outre au moins un agent de bronzage et/ou de brunissage artificiel de la peau.

18. Composition selon l'une quelconque des revendications 1 à 17, **caractérisée par le fait qu'**elle comprend en outre au moins un adjuvant choisi parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants

19. Composition selon l'une quelconque des revendications 1 à 18, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une huile, d'une poudre, d'un bâtonnet solide, d'une mousse ou d'un spray.

20. Composition selon l'une quelconque des revendications 1 à 18, **caractérisée par le fait qu'**il s'agit d'une composition de maquillage des cils, des sourcils, des ongles ou de la peau et qu'elle se présente sous forme solide ou pâteuse, anhydre ou aqueuse, d'une émulsion, d'une suspension ou d'une dispersion.

21. Composition selon l'une quelconque des revendications 1 à 18, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

22. Utilisation d'une composition telle que définie dans les revendications 1 à 21 pour la fabrication de compositions cosmétiques ou dermatologiques destinées à la protection de la peau et/ou des cheveux contre le rayonnement ultraviolet, en particulier le rayonnement solaire.

23. Procédé pour améliorer la stabilité d'au moins un dérivé du dibenzoylméthane vis-à-vis du rayonnement UV, **caractérisé par le fait qu'**il consiste à associer au dit dérivé du dibenzoylméthane une quantité efficace de dérivé de 3-(2-azacycloalkylidene)-1,3-dihydro-indol-2-one tel que défini dans l'une quelconque des revendications précédentes.

24. Utilisation d'un dérivé de 3-(2-azacycloalkylidene)-1,3-dihydro-indol-2-one tel que défini dans l'une quelconque des revendications précédentes dans la préparation d'une composition cosmétique ou dermatologique comprenant au moins un filtre UV du type dérivé du dibenzoylméthane dans le but d'améliorer la stabilité vis-à-vis des rayons UV dudit dérivé du dibenzoylméthane.
